(19) Europäisches Patentamt
European Patent Office
Office européen des brevets

(11) EP 4 567 816 A1

(12) **EUROPEAN PATENT APPLICATION**
published in accordance with Art. 153(4) EPC

(43) Date of publication:
**11.06.2025 Bulletin 2025/24**

(21) Application number: **23860222.1**

(22) Date of filing: **25.08.2023**

(51) International Patent Classification (IPC):
**G16B 15/30** (2019.01)

(52) Cooperative Patent Classification (CPC):
**G16B 15/30**

(86) International application number:
**PCT/JP2023/030775**

(87) International publication number:
**WO 2024/048460 (07.03.2024 Gazette 2024/10)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **01.09.2022 JP 2022139395**

(71) Applicant: **Kagoshima University
Kagoshima-shi, Kagoshima 890-8580 (JP)**

(72) Inventors:
• **ISHIKAWA Takeshi
Kagoshima-shi, Kagoshima 890-8580 (JP)**
• **OZONO Hiroki
Kagoshima-shi, Kagoshima 890-8580 (JP)**

(74) Representative: **v. Bezold & Partner
Patentanwälte - PartG mbB
Ridlerstraße 57
80339 München (DE)**

(54) **INTERMOLECULAR INTERACTION ANALYSIS DEVICE, INTERMOLECULAR INTERACTION ANALYSIS METHOD, AND PROGRAM**

(57) An intermolecular interaction analysis device (1) includes a contact surface definer (30) that defines a surface where a partial electron density of molecule A calculated by an electron density calculator (10) and a partial electron density of molecule B calculated by an electron density calculator (20) are equal as a contact surface, an electrostatic potential calculator (40) that calculates a partial electrostatic potential of molecule A at the contact surface by an FMO method while excluding a contribution of charged amino acid residues corresponding to the charge of molecule A, and an electrostatic potential calculator (50) that calculates a partial electrostatic potential of molecule B at the contact surface by an FMO method while excluding a contribution of charged amino acid residues corresponding to the charge of molecule B.

FIG. 1

## Description

Technical Field

The present disclosure relates to an intermolecular interaction analysis device, an intermolecular interaction analysis method, and a program.

Background Art

Based on the three-dimensional (3D) structure data of the ligand-binding site of a protein that is a target for drug discovery and the 3D structure data of a compound, the intermolecular interaction between the ligand-binding site and the compound can be evaluated. By evaluating the intermolecular interaction using computational chemistry, it is possible to design a compound that can bind the ligand-binding site with high affinity. It is also possible to perform virtual screening to comprehensively evaluate many compounds.

For example, Patent Literature 1 discloses a protein interaction analysis device that generates data on an interaction using surface shape data of a ligand-binding site, electrostatic potential distribution data of the ligand-binding site, and 3D structure data regarding a ligand interacting with the ligand-binding site.

Many biological functions, such as signal transduction, transport, and metabolism in vivo, are controlled by a protein-protein interaction (PPI), which collectively refers to an interaction between protein molecules. An antibody that binds to a target protein via PPI has high affinity and specificity and is therefore used as a pharmaceutical. The development of antibody drugs has increased in recent years because antibodies can target proteins that cannot be targeted by small molecule compounds.

For example, when programmed death-ligand 1 (PD-L1) on a cancer cell binds to programmed death-1 (PD-1) on a T cell, cytokine production from the T cell is reduced, and a signal that suppresses the activity of the T cell is transmitted. The contact surface between PD-1 and PD-L1 is nearly flat and lacks a 3D pocket-like structure, making inhibition by small molecule compounds difficult. Meanwhile, nivolumab, an antibody drug, binds to PD-1 via PPI and can inhibit the binding between PD-1 and PD-L1. Thus, a detailed understanding of PPI is important for elucidating biological functions and developing antibody drugs.

In Non Patent Literature 1, in a protein complex, the partial electron density of each protein is calculated by the fragment molecular orbital method, and a surface on which each partial electron density is the same is defined as a contact surface. Furthermore, Non Patent Literature 1 discloses a technique for neutralizing the partial electrostatic potential at the contact surface by adding ions so as to cancel the electric charge of each protein. This allows estimation of the charge distribution of both molecules at the binding interface and visualization of the electrostatic interactions.

Citation List

Patent Literature

Patent Literature 1: International Publication No. WO 2019/235567

Non Patent Literature

Non Patent Literature 1: H. Ozono and T. Ishikawa, "Visualization of the Interfacial Electrostatic Complementarity: A Method for Analysis of Protein-Protein Interaction Based on Ab Initio Quantum Chemical Calculations," J Chem. Theory Comput., 2021, 17, 5600-5610

Summary of Invention

Technical Problem

Analysis of intermolecular interactions by computational chemistry, such as the protein interaction analysis device disclosed in Patent Literature 1, has been applied primarily to the analysis of interactions between proteins and low molecular weight compounds. In the analysis of PPIs, a large contact surface must be taken into consideration, and complex interactions formed by a plurality of amino acid residues must be analyzed. It is difficult to analyze PPIs with high accuracy using existing computational chemistry methods for analyzing intermolecular interactions.

Moreover, in Non Patent Literature 1, the calculated results depend on the type or position of the added ion, resulting in a decrease in accuracy, and the amount of calculation increases because an additional calculation with the addition of the ion must be performed. Thus, further improvement is required.

The present disclosure has been made in view of the above-described circumstances. An objective of the disclosure is to provide an intermolecular interaction analysis device, an intermolecular interaction analysis method, and a program which can evaluate interactions between molecules including a plurality of amino acid residues with high accuracy.

Solution to Problem

An intermolecular interaction analysis device of a first aspect of the present disclosure includes:

a first electron density calculator that calculates a partial electron density of a first molecule containing a plurality of amino acid residues linked via peptide bonds regarding 3D structure data of the first molecule by a fragment molecular orbital method;
a second electron density calculator that calculates a partial electron density of a second molecule containing a plurality of amino acid residues linked via peptide bonds, the second molecule being arranged at a position interacting with the first molecule relative to a position of the first molecule, regarding 3D structure data of the second molecule by a fragment molecular orbital method;
a contact surface definer that defines a surface where the partial electron density of the first molecule and the partial electron density of the second molecule are equal as a contact surface;
a first electrostatic potential calculator that calculates a partial electrostatic potential of the first molecule at the contact surface by a fragment molecular orbital method while, with the first molecule having a charge, excluding a contribution of charged amino acid residues corresponding to the charge of the first molecule; and
a second electrostatic potential calculator that calculates a partial electrostatic potential of the second molecule at the contact surface by a fragment molecular orbital method while, with the second molecule having a charge, excluding a contribution of charged amino acid residues corresponding to the charge of the second molecule.

The contact surface definer may define a surface where the partial electron density of the first molecule is equal to or greater than a reference value as the contact surface.
The first electrostatic potential calculator may,

with the first molecule having a positive charge of +n, select n residues among arginine residues and lysine residues contained in the first molecule as the charged amino acid residues in descending order of distance from the contact surface, and calculate a partial electrostatic potential of the first molecule as a whole while excluding a partial electrostatic potential of the selected charged amino acid residues, or
with the first molecule having a negative charge of -n, select n residues among aspartic acid and glutamic acid residues contained in the first molecule as the charged amino acid residues in descending order of distance from the contact surface, and calculate a partial electrostatic potential of the first molecule as a whole while excluding a partial electrostatic potential of the selected charged amino acid residues, and
the second electrostatic potential calculator may,
with the second molecule having a positive charge of +n, select n residues among arginine residues and lysine residues contained in the second molecule as the charged amino acid residues in descending order of distance from the contact surface, and calculate a partial electrostatic potential of the second molecule as a whole while excluding a partial electrostatic potential of the selected charged amino acid residues, or
with the second molecule having a negative charge of -n, select n residues among aspartic acid and glutamic acid residues contained in the second molecule as the charged amino acid residues in descending order of distance from the contact surface, and calculate a partial electrostatic potential of the second molecule as a whole while excluding a partial electrostatic potential of the selected charged amino acid residues.

An intermolecular interaction analysis device of a second aspect of the present disclosure includes:

a first electron density calculator that calculates a partial electron density of a first molecule containing a plurality of amino acid residues linked via peptide bonds regarding 3D structure data of the first molecule by a fragment molecular orbital method;
a second electron density calculator that calculates a partial electron density of a second molecule containing a plurality of amino acid residues linked via peptide bonds, the second molecule being arranged at a position interacting with the first molecule relative to a position of the first molecule, regarding 3D structure data of the second molecule by a fragment molecular orbital method;
a contact surface definer that defines a surface where the partial electron density of the first molecule and the partial electron density of the second molecule are equal as a contact surface; and

a dispersion force evaluator that evaluates dispersion force based on a partial electron density of the first molecule and a partial electron density of the second molecule at the contact surface.

The contact surface definer may define a surface where the partial electron density of the first molecule is equal to or greater than a reference value as the contact surface.

The dispersion force evaluator may evaluate an index indicating an overlap of partial electron densities calculated from a sum of the partial electron density of the first molecule and the partial electron density of the second molecule at the contact surface as the dispersion force.

An intermolecular interaction analysis device of a third aspect of the present disclosure includes:

a first electron density calculator that calculates a partial electron density of a first molecule containing a plurality of amino acid residues linked via peptide bonds regarding 3D structure data of the first molecule by a fragment molecular orbital method;

a second electron density calculator that calculates a partial electron density of a second molecule containing a plurality of amino acid residues linked via peptide bonds, the second molecule being arranged at a position interacting with the first molecule relative to a position of the first molecule, regarding 3D structure data of the second molecule by a fragment molecular orbital method;

a contact surface definer that defines a surface where the partial electron density of the first molecule and the partial electron density of the second molecule are equal as a contact surface;

a first electrostatic potential calculator that calculates a partial electrostatic potential of the first molecule at the contact surface by a fragment molecular orbital method while, with the first molecule having a charge, excluding a contribution of charged amino acid residues corresponding to the charge of the first molecule;

a second electrostatic potential calculator that calculates a partial electrostatic potential of the second molecule at the contact surface by a fragment molecular orbital method while, with the second molecule having a charge, excluding a contribution of charged amino acid residues corresponding to the charge of the second molecule; and

a dispersion force evaluator that evaluates dispersion force based on a partial electron density of the first molecule and a partial electron density of the second molecule at the contact surface.

An intermolecular interaction analysis method of a fourth aspect of the present disclosure includes:

calculating a partial electron density of a first molecule containing a plurality of amino acid residues linked via peptide bonds regarding 3D structure data of the first molecule by a fragment molecular orbital method;

calculating a partial electron density of a second molecule containing a plurality of amino acid residues linked via peptide bonds, the second molecule being arranged at a position interacting with the first molecule relative to a position of the first molecule, regarding 3D structure data of the second molecule by a fragment molecular orbital method;

defining a surface where the partial electron density of the first molecule and the partial electron density of the second molecule are equal as a contact surface;

calculating a partial electrostatic potential of the first molecule at the contact surface by a fragment molecular orbital method while, with the first molecule having a charge, excluding a contribution of charged amino acid residues corresponding to the charge of the first molecule; and

calculating a partial electrostatic potential of the second molecule at the contact surface by a fragment molecular orbital method while, with the second molecule having a charge, excluding a contribution of charged amino acid residues corresponding to the charge of the second molecule.

An intermolecular interaction analysis method of a fifth aspect of the present disclosure includes:

calculating a partial electron density of a first molecule containing a plurality of amino acid residues linked via peptide bonds regarding 3D structure data of the first molecule by a fragment molecular orbital method;

calculating a partial electron density of a second molecule containing a plurality of amino acid residues linked via peptide bonds, the second molecule being arranged at a position interacting with the first molecule relative to a position of the first molecule, regarding 3D structure data of the second molecule by a fragment molecular orbital method;

defining a surface where the partial electron density of the first molecule and the partial electron density of the second molecule are equal as a contact surface; and

evaluating dispersion force based on a partial electron density of the first molecule and a partial electron density of the second molecule at the contact surface.

An intermolecular interaction analysis method of a sixth aspect of the present disclosure includes:

calculating a partial electron density of a first molecule containing a plurality of amino acid residues linked via peptide bonds regarding 3D structure data of the first molecule by a fragment molecular orbital method;

calculating a partial electron density of a second molecule containing a plurality of amino acid residues linked via peptide bonds, the second molecule being arranged at a position interacting with the first molecule relative to a position of the first molecule, regarding 3D structure data of the second molecule by a fragment molecular orbital method;

defining a surface where the partial electron density of the first molecule and the partial electron density of the second molecule are equal as a contact surface;

calculating a partial electrostatic potential of the first molecule at the contact surface by a fragment molecular orbital method while, with the first molecule having a charge, excluding a contribution of charged amino acid residues corresponding to the charge of the first molecule;

calculating a partial electrostatic potential of the second molecule at the contact surface by a fragment molecular orbital method while, with the second molecule having a charge, excluding a contribution of charged amino acid residues corresponding to the charge of the second molecule; and

evaluating dispersion force based on a partial electron density of the first molecule and a partial electron density of the second molecule at the contact surface.

A program of a seventh aspect of the present disclosure allows a computer to function as:

a first electron density calculator that calculates a partial electron density of a first molecule containing a plurality of amino acid residues linked via peptide bonds regarding 3D structure data of the first molecule by a fragment molecular orbital method;

a second electron density calculator that calculates a partial electron density of a second molecule containing a plurality of amino acid residues linked via peptide bonds, the second molecule being arranged at a position interacting with the first molecule relative to a position of the first molecule, regarding 3D structure data of the second molecule by a fragment molecular orbital method;

a contact surface definer that defines a surface where the partial electron density of the first molecule and the partial electron density of the second molecule are equal as a contact surface;

a first electrostatic potential calculator that calculates a partial electrostatic potential of the first molecule at the contact surface by a fragment molecular orbital method while, with the first molecule having a charge, excluding a contribution of charged amino acid residues corresponding to the charge of the first molecule; and

a second electrostatic potential calculator that calculates a partial electrostatic potential of the second molecule at the contact surface by a fragment molecular orbital method while, with the second molecule having a charge, excluding a contribution of charged amino acid residues corresponding to the charge of the second molecule.

A program of an eighth aspect of the present disclosure allows a computer to function as:

a first electron density calculator that calculates a partial electron density of a first molecule containing a plurality of amino acid residues linked via peptide bonds regarding 3D structure data of the first molecule by a fragment molecular orbital method;

a second electron density calculator that calculates a partial electron density of a second molecule containing a plurality of amino acid residues linked via peptide bonds, the second molecule being arranged at a position interacting with the first molecule relative to a position of the first molecule, regarding 3D structure data of the second molecule by a fragment molecular orbital method;

a contact surface definer that defines a surface where the partial electron density of the first molecule and the partial electron density of the second molecule are equal as a contact surface; and

a dispersion force evaluator that evaluates dispersion force based on a partial electron density of the first molecule and a partial electron density of the second molecule at the contact surface.

A program of a ninth aspect of the present disclosure allows a computer to function as:

a first electron density calculator that calculates a partial electron density of a first molecule containing a plurality of amino acid residues linked via peptide bonds regarding 3D structure data of the first molecule by a fragment molecular orbital method;

a second electron density calculator that calculates a partial electron density of a second molecule containing a plurality of amino acid residues linked via peptide bonds, the second molecule being arranged at a position interacting with the first molecule relative to a position of the first molecule, regarding 3D structure data of the second molecule by a fragment molecular orbital method;

a contact surface definer that defines a surface where the partial electron density of the first molecule and the partial electron density of the second molecule are equal as a contact surface; and

a first electrostatic potential calculator that calculates a partial electrostatic potential of the first molecule at the contact surface by a fragment molecular orbital method while, with the first molecule having a charge, excluding a contribution of charged amino acid residues corresponding to the charge of the first molecule;

a second electrostatic potential calculator that calculates a partial electrostatic potential of the second molecule at the contact surface by a fragment molecular orbital method while, with the second molecule having a charge, excluding a contribution of charged amino acid residues corresponding to the charge of the second molecule; and

a dispersion force evaluator that evaluates dispersion force based on a partial electron density of the first molecule and a partial electron density of the second molecule at the contact surface.

An intermolecular interaction analysis device of a tenth aspect of the present disclosure includes:

a memory that stores a program; and
a central processing unit (CPU) that reads and executes the program from the memory,
wherein the CPU
calculates a partial electron density of a first molecule containing a plurality of amino acid residues linked via peptide bonds regarding 3D structure data of the first molecule by a fragment molecular orbital method,
calculates a partial electron density of a second molecule containing a plurality of amino acid residues linked via peptide bonds, the second molecule being arranged at a position interacting with the first molecule relative to a position of the first molecule, regarding 3D structure data of the second molecule by a fragment molecular orbital method,
defines a surface where the partial electron density of the first molecule and the partial electron density of the second molecule are equal as a contact surface,
calculates a partial electrostatic potential of the first molecule at the contact surface by a fragment molecular orbital method while, with the first molecule having a charge, excluding a contribution of charged amino acid residues corresponding to the charge of the first molecule, and
calculates a partial electrostatic potential of the second molecule at the contact surface by a fragment molecular orbital method while, with the second molecule having a charge, excluding a contribution of charged amino acid residues corresponding to the charge of the second molecule.

An intermolecular interaction analysis device of an eleventh aspect of the present disclosure includes:

a memory that stores a program; and
a central processing unit (CPU) that reads and executes the program from the memory,
wherein the CPU
calculates a partial electron density of a first molecule containing a plurality of amino acid residues linked via peptide bonds regarding 3D structure data of the first molecule by a fragment molecular orbital method,
calculates a partial electron density of a second molecule containing a plurality of amino acid residues linked via peptide bonds, the second molecule being arranged at a position interacting with the first molecule relative to a position of the first molecule, regarding 3D structure data of the second molecule by a fragment molecular orbital method,
defines a surface where the partial electron density of the first molecule and the partial electron density of the second molecule are equal as a contact surface, and
evaluates dispersion force based on a partial electron density of the first molecule and a partial electron density of the second molecule at the contact surface.

An intermolecular interaction analysis device of a twelfth aspect of the present disclosure includes:

a memory that stores a program; and
a central processing unit (CPU) that reads and executes the program from the memory,
wherein the CPU
calculates a partial electron density of a first molecule containing a plurality of amino acid residues linked via peptide bonds regarding 3D structure data of the first molecule by a fragment molecular orbital method,
calculates a partial electron density of a second molecule containing a plurality of amino acid residues linked via peptide bonds, the second molecule being arranged at a position interacting with the first molecule relative to a position of the first molecule, regarding 3D structure data of the second molecule by a fragment molecular orbital method,

defines a surface where the partial electron density of the first molecule and the partial electron density of the second molecule are equal as a contact surface,

calculates a partial electrostatic potential of the first molecule at the contact surface by a fragment molecular orbital method while, with the first molecule having a charge, excluding a contribution of charged amino acid residues corresponding to the charge of the first molecule,

calculates a partial electrostatic potential of the second molecule at the contact surface by a fragment molecular orbital method while, with the second molecule having a charge, excluding a contribution of charged amino acid residues corresponding to the charge of the second molecule, and

evaluates dispersion force based on a partial electron density of the first molecule and a partial electron density of the second molecule at the contact surface.

Advantageous Effects of Invention

According to the present disclosure, interactions between molecules including a plurality of amino acid residues can be evaluated with high accuracy.

Brief Description of Drawings

FIG. 1 is a diagram illustrating a configuration of an intermolecular interaction analysis device according to Embodiment 1 of the present disclosure;

FIG. 2 is a diagram illustrating the concept of partial electron density calculation range and the concept of 3D grid data;

FIG. 3A is a diagram illustrating the partial electrostatic potential of nivolumab as an example of a contact surface with added information regarding the partial electrostatic potential;

FIG. 3B is a diagram illustrating the partial electrostatic potential of PD-1 as an example of a contact surface with added information regarding the partial electrostatic potential;

FIG. 4 is a block diagram illustrating the hardware configuration of the intermolecular interaction analysis device of FIG. 1;

FIG. 5 is a diagram illustrating a flowchart of electrostatic potential evaluation processing performed by the intermolecular interaction analysis device of FIG. 1;

FIG. 6 is a diagram illustrating a configuration of an intermolecular interaction analysis device according to Embodiment 2 of the present disclosure;

FIG. 7 is a diagram illustrating an example of a contact surface to which an index indicating an overlap of partial electron densities is added;

FIG. 8 is a block diagram illustrating the hardware configuration of the intermolecular interaction analysis device of FIG. 6; and

FIG. 9 is a diagram illustrating a flowchart of dispersion force evaluation processing performed by the intermolecular interaction analysis device of FIG. 6.

Description of Embodiments

Hereinafter, embodiments of the present disclosure are described with reference to the drawings. In each drawing, the same or equivalent parts are given the same reference numerals. The present disclosure is not limited to the following embodiments and drawings. In the following embodiments, the terms "have," "include," and "contain" also encompass the meanings "consist of" and "comprise."

Embodiment 1

Embodiment 1 of the present disclosure is described. The intermolecular interaction analysis device according to the present embodiment serving as an information processor analyzes an intermolecular interaction between a molecule A (first molecule) and a molecule B (second molecule). The molecule A and the molecule B each include a plurality of amino acid residues linked via peptide bonds. Examples of molecules A and B include proteins, peptides, and antibodies. The molecules A and B may be molecules that contain a plurality of amino acid chains linked by bonds other than peptide bonds, such as disulfide bonds, like antibodies. The intermolecular interaction encompasses electrostatic interaction, van der Waals force, dipole-dipole interaction, dispersion force, hydrogen bond, hydrophobic interaction, and the like acting between molecules A and B.

The intermolecular interaction analysis device according to the present embodiment is constructed on a computer. In other words, the intermolecular interaction analysis device is an information processor in which software-based information processing is specifically realized using hardware resources.

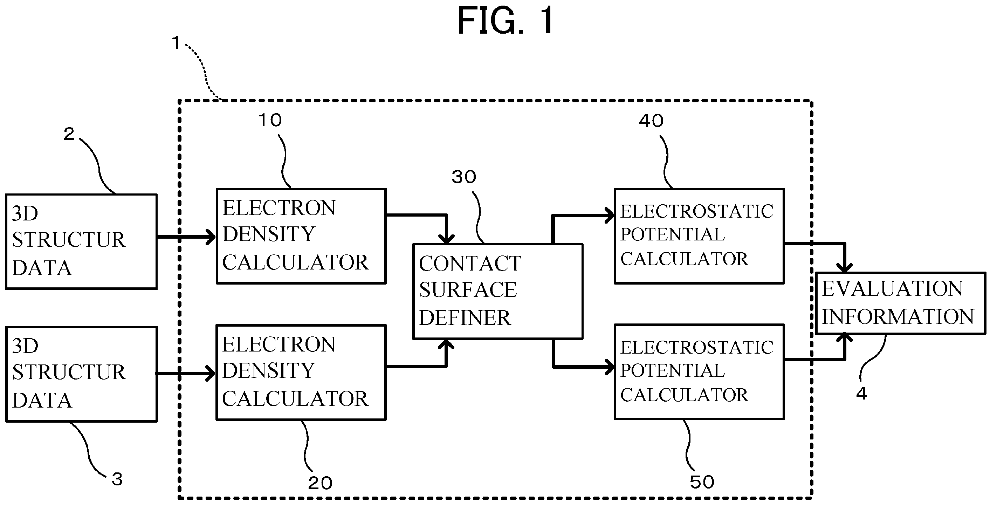

As illustrated in FIG. 1, an intermolecular interaction analysis device 1 receives 3D structure data 2 of a molecule A and 3D structure data 3 of a molecule B. The 3D structure data 2 is information indicating the position in the 3D space of each atom constituting the molecule A, and specifically, is coordinate data of each atom constituting the molecule A determined by X-ray crystal structure analysis, NMR, or the like. The 3D structure data 2 can be obtained from the Protein Data Bank (PDB) or the like. In the case of using coordinate data obtained from the PDB as the 3D structure data 2, water molecules may be deleted from the coordinate data, hydrogen atoms may be added to the 3D structure, and the N-terminus and C-terminus may be capped with - $COCH_3$ and -$NHCH_3$, respectively. Coordinate data of a 3D structure optimized using a known force field may be used as the 3D structure data 2. The 3D structure data 3 is similar to the 3D structure data 2.

The intermolecular interaction analysis device 1 performs calculations on the 3D structure data 2 and the 3D structure data 3, and outputs evaluation information 4 regarding the intermolecular interaction between the molecules A and B. The evaluation information 4 includes the magnitude of the electrostatic potential contributing to the interaction between molecules A and B, as well as the degree of correspondence between the electrostatic potential of molecule A and the electrostatic potential of molecule B.

The configuration of the intermolecular interaction analysis device 1 is described in more detail. The intermolecular interaction analysis device 1 comprises an electron density calculator 10, an electron density calculator 20, a contact surface definer 30, an electrostatic potential calculator 40, and an electrostatic potential calculator 50 as illustrated in FIG. 1.

The electron density calculator 10 calculates the partial electron density of molecule A using the 3D structure data 2 of molecule A by the fragment molecular orbital (FMO) method. The FMO method is a method of calculating the total energy and inter-fragment interaction energy (IFIE) of a molecule by dividing a macromolecular system such as a protein or a polymer into small fragments of several tens of atoms and repeatedly performing calculations based on the *ab initio* molecular orbital method for individual fragments (monomers) and fragment pairs (dimers). For the FMO method, a known software program such as PAICS can be used.

Here, the calculation of the electron density by the FMO method is described. First, a protein is divided into fragments of individual amino acid residues to divide the protein into smaller fragments. Since monomer and dimer calculations must take into account the electrostatic potential based on the electron density of the surrounding fragments, a monomer self-consistent charge (SCC) calculation is performed to determine the electron density of each fragment. In the monomer SCC calculation, an initial electron density is determined, and a monomer calculation of each fragment is performed in the presence of an electrostatic potential due to this initial electron density. Once calculations are completed for all fragments, a new electron density is obtained that is different from the initial electron density. Next, the new electron density is used as the initial electron density, and the calculation is carried out in the same manner. This process is repeated until a self-consistent electron density is obtained, which is the final electron density. Monomer and dimer calculations are performed taking into account the electrostatic potential created by this final electron density. Finally, the overall properties are calculated from only the results of the monomer and dimer calculations. The total energy is calculated using the monomer energy ($E_I$) and the dimer energy ($E_{IJ}$) as shown in Formula 1.

[Formula 1]

$$E_{total} = \sum_{I<J} E_{IJ} - (N-2) \sum_{I} E_I$$

Formula 1 can be replaced with the following Formula 2.

[Formula 2]

$$E_{total} = \sum_{I} E_I + \sum_{I<J} \Delta E_{IJ}$$

The first term on the right-hand side of Formula 2 is the energy of the monomer excluding electrostatic potential from other fragments. The second term on the right-hand side represents the interaction energy between fragments, IFIE.

The electron density is also calculated by the FMO method, and is obtained from the electron density of the monomer ($\rho_I(r)$) and the electron density of the dimer ($\rho_{IJ}(r)$) at position r as shown in Formula 3.

[Formula 3]

$$\rho_{total}(r) = \sum_I \rho_I(r) + \sum_{I<J} \Delta\rho_{IJ}(r)$$

Returning to FIG. 1, the electron density calculator 20 calculates the partial electron density of molecule B by the FMO method for the 3D structure data 3 of a molecule B arranged at a position interacting with a molecule A relative to the position of the molecule A.

The contact surface definer 30 defines a contact surface from the partial electron density of molecule A and the partial electron density of molecule B. In a complex of molecules A and B, the electron density of the entire complex ($\rho_{total}(r)$) can be divided into the partial electron density of molecule A ($\rho^{part\text{-}A}(r)$) and the partial electron density of molecule B ($\rho^{part\text{-}B}(r)$) and the partial electron density of molecule A and the partial electron density of molecule B can be calculated by Formula 4 and Formula 5 respectively.

[Formula 4]

$$\rho^{part\text{-}A}(r) = \sum_{I\in A} \rho_I(r) + \sum_{I<J\in A} \Delta\rho_{IJ}(r) + \frac{1}{2}\sum_{I\in A}\sum_{J\in B} \Delta\rho_{IJ}(r)$$

[Formula 5]

$$\rho^{part\text{-}B}(r) = \sum_{I\in B} \rho_I(r) + \sum_{I<J\in B} \Delta\rho_{IJ}(r) + \frac{1}{2}\sum_{I\in A}\sum_{J\in B} \Delta\rho_{IJ}(r)$$

[Formula 6]

$$(\rho_{total}(r)) = (\rho^{part\text{-}A}(r)) + (\rho^{part\text{-}B}(r))$$

In Formula 4 for the partial electron density of molecule A, the first term on the right-hand side is the contribution of the monomer in a molecule A, and the second term is the contribution of the dimer in a molecule A. The third term represents the contribution of the dimer between molecules A and B. The same is true for Formula 5 for the partial electron density of molecule B. The partial electron density of molecule A and the partial electron density of molecule B combine to give the electron density of the entire complex, as shown in Formula 6.

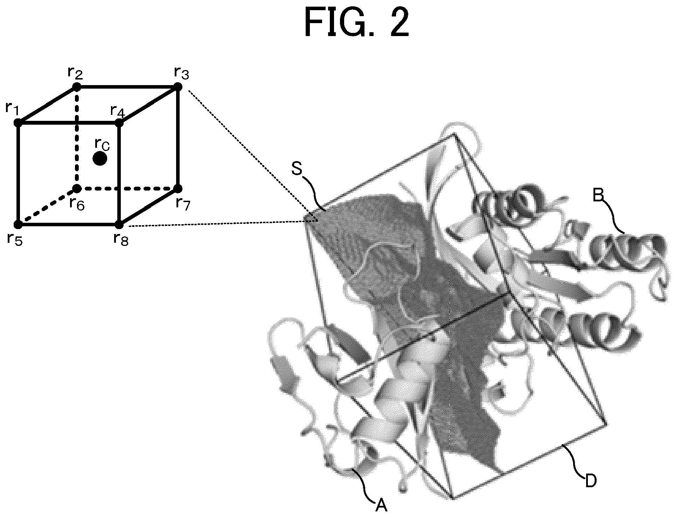

For example, the partial electron density is calculated using 3D grid data. First, a range D for setting a grid lattice is set for molecules A and B illustrated in FIG. 2. The range D is set by any method so as to include amino acid residues that contribute to the interaction between molecules A and B. For example, the range D includes amino acid residues in a molecule A within a distance L from a molecule B, and amino acid residues in the molecule B within a distance L from the molecule A. The distance L is appropriately set in consideration of the sizes of molecules A and B, the size of the range of interaction, and the like. Next, the range D is divided into cubes, each of which is a grid. The length of one side of the grid lattice is appropriately set, for example, to 0.3 Å. As illustrated in FIG. 2, with a focus on one grid cell consisting of eight points $r_1$ to $r_8$, the electron density calculator 10 calculates the partial electron density at $r_1$ to $r_8$ for the molecule A. The partial electron density of the grid lattice calculated for the molecule A (the partial electron density of molecule A) is obtained as the average value of the partial electron densities of $r_1$ to $r_8$ at the center point $r_c$ of the grid lattice. Meanwhile, similarly, the partial electron density of the grid lattice (the partial electron density of molecule B) calculated for the molecule B by the electron density calculator 20 is obtained.

The contact surface definer 30 defines a surface where the partial electron density of molecule A and the partial electron density of molecule B are equal to a contact surface S. In the above example, in a case in which the partial electron density of molecule A and the partial electron density of molecule B are equal, the point $r_c$ becomes a point that constitutes a contact surface S. Here, since the size of the contact surface S depends on the size of the area D, it is preferable that the contact surface definer 30 defines a surface where the partial electron density of molecule A is equal to or greater than a

reference value $\rho_c$ as a contact surface S. $\rho_c$ is, for example, $10^{-4}$ atomic units (a.u.) or more.

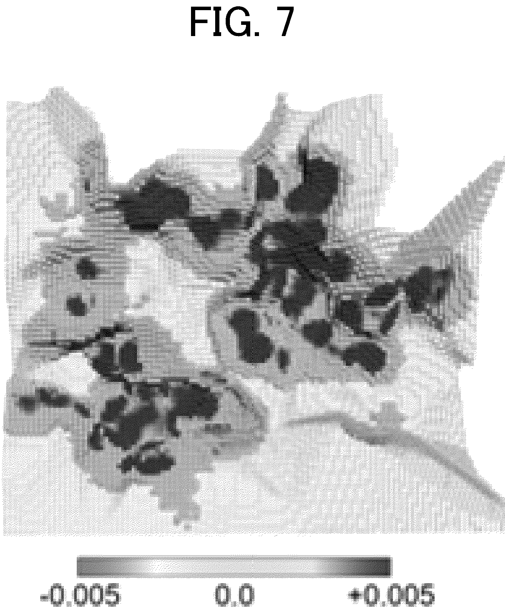

Returning to FIG. 1, the electrostatic potential calculator 40 calculates the partial electrostatic potential of molecule A at the contact surface S by the FMO method. Here, the calculation of the electrostatic potential by the FMO method is described. The electrostatic potential of the entire complex consisting of molecules A and B ($\varphi_{total}(r)$) can be calculated from the electrostatic potential of the monomer ($\varphi_I(r)$) and the electrostatic potential of the dimer ($\varphi_{IJ}(r)$) at position r, atomic position $R_\alpha$, and nuclear charge $Z_\alpha$ as illustrated in FIG. 7.

[Formula 7]

$$\phi_{total}(r) = \sum_I \phi_I(r) + \sum_{I<J} \Delta\phi_{IJ}(r) + \sum_\alpha \frac{Z_\alpha}{|r - R_\alpha|}$$

$\varphi_{total}(r)$ can be divided into the partial electrostatic potential of molecule A ($\varphi^{part-A}(r)$) and the partial electrostatic potential of molecule B ($\varphi^{part-B}(r)$). Thus, the partial electrostatic potential of molecule A and the partial electrostatic potential of molecule B are calculated from Formulas 8 and 9, respectively.

[Formula 8]

$$\phi^{part-A}(r) = \sum_{I \in A} \phi_I(r) + \sum_{I<J \in A} \Delta\phi_{IJ}(r) + \sum_{\alpha \in A} \frac{Z_\alpha}{|r - R_\alpha|} + \frac{1}{2}\sum_{I \in A}\sum_{J \in B} \Delta\phi_{IJ}(r)$$

[Formula 9]

$$\phi^{part-B}(r) = \sum_{I \in B} \phi_I(r) + \sum_{I<J \in B} \Delta\phi_{IJ}(r) + \sum_{\alpha \in B} \frac{Z_\alpha}{|r - R_\alpha|} + \frac{1}{2}\sum_{I \in A}\sum_{J \in B} \Delta\phi_{IJ}(r)$$

[Formula 10]

$$(\varphi_{total}(r)) = (\varphi^{part-A}(r)) + (\varphi^{part-B}(r))$$

In Formula 8, for the partial electrostatic potential of a molecule A, the first term on the right-hand side is the contribution of the monomer in the molecule A, and the second term is the contribution of the dimer in the molecule A. The third term is the contribution due to the nuclei in the molecule A. The fourth term represents the contribution of the dimer between molecules A and B. The same is true for Formula 9 for the partial electrostatic potential of molecule B. The partial electrostatic potential of molecule A and the partial electrostatic potential of molecule B combine to give the electrostatic potential of the entire complex, as shown in Formula 10.

In a case in which the molecule A has a charge, a bias in the partial electrostatic potential at the contact surface S may occur. Even in the case of a charged protein, the protein is neutralized *in vivo* by counter ions present in a solution. In order to suppress the effect of the total charge while maintaining the original partial electrostatic potential state at the contact surface S, in a case in which the molecule A has a charge, the electrostatic potential calculator 40 calculates a partial electrostatic potential of molecule A at the contact surface S by an FMO method while excluding a contribution of charged amino acid residues corresponding to the charge of molecule A. More specifically, in a case in which the molecule A has a positive charge of +n, the electrostatic potential calculator 40 selects n residues among arginine residues and lysine residues contained in the molecule A in descending order of distance from the contact surface S as charged amino acid residues. Then, the electrostatic potential calculator 40 calculates the partial electrostatic potential of the molecule A as a whole while excluding the partial electrostatic potential of the selected charged amino acid residue. Meanwhile, in a case in which the molecule A has a negative charge of -n, the electrostatic potential calculator 40 selects n residues among aspartic acid and glutamic acid residues contained in the molecule A in descending order of distance from the contact surface S as charged amino acid residues. The electrostatic potential calculator 40 calculates the partial electrostatic potential of the molecule A as a whole while excluding the partial electrostatic potential of the selected charged amino acid residues. When the charge of the molecule A is 0, the electrostatic potential calculator 40 calculates the partial electrostatic potential of the molecule A at the contact surface S without excluding the partial electrostatic potential of the charged amino acid residues.

For example, in a case in which the molecule A has a charge of -3, the electrostatic potential calculator 40 calculates a distance between each of aspartic acid and glutamic acid residues contained in the molecule A and the contact surface S, ranks the residues in descending order of distance, and excludes the partial electrostatic potentials of the top three charged amino acid residues, thereby calculating the partial electrostatic potential of the molecule A. Conversely, in a case in which the molecule A has a charge of +2, the electrostatic potential calculator 40 calculates a distance between each of arginine or lysine residues contained in the molecule A and the contact surface S, ranks the residues in descending order of distance, and excludes the partial electrostatic potentials of the top two charged amino acid residues, thereby calculating the partial electrostatic potential of the molecule A.

The distance between the contact surface S and the charged amino acid residues may be calculated as the distance from the nearest atom of the molecule B to the charged amino acid residues of the molecule A. In addition, when calculating the distance from the contact surface S or atom to the charged amino acid residue, the position of the $\alpha$ carbon may be used as the position of the charged amino acid residue, or the average value of the coordinates of all atoms constituting the charged amino acid residues may be used.

The electrostatic potential calculator 50 calculates the partial electrostatic potential of molecule B at the contact surface S by the FMO method, as with the electrostatic potential calculator 40. When the molecule B has a charge, the electrostatic potential calculator 50 calculates a partial electrostatic potential of molecule B at the contact surface S by an FMO method while excluding a contribution of charged amino acid residues corresponding to the charge of molecule B, as with the electrostatic potential calculator 40.

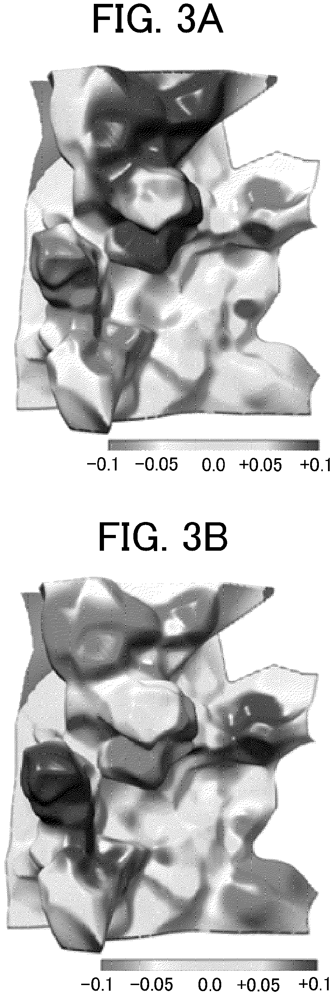

The partial electrostatic potential of molecule A and the partial electrostatic potential of molecule B obtained as the evaluation information 4 using the electrostatic potential calculator 40 and the electrostatic potential calculator 50 can be displayed on the contact surface S by, for example, color-coding the potentials according to their respective values. For example, FIG. 3A illustrates the partial electrostatic potential of nivolumab at the contact surface S for a complex of PD-1 and the antibody nivolumab that binds to PD-1. FIG. 3B illustrates the partial electrostatic potential of PD-1 at the contact surface S for a complex of PD-1 and the antibody nivolumab that binds to PD-1. Comparing FIGS. 3A and 3B, the electrical positivity and negativity at the contact surface S almost correspond to each other, indicating the high electrical affinity between PD-1 and nivolumab. For example, the electrostatic potential calculator 40 calculates the sum of the attractive interactions (opposite signs) of the grid lattice of the contact surface S and the sum $\varphi^-$ and the repulsive interactions (same signs) of the grid lattice of the contact surface S, $\varphi^+$, and calculates the ratio $W^-$ of $\varphi^-$ to the sum of $\varphi^-$ and $\varphi^+$ as evaluation information 4. $W^-$ can be used as an index of the degree of correspondence (electrostatic complementarity) between the electrostatic potential of molecule A and the electrostatic potential of molecule B.

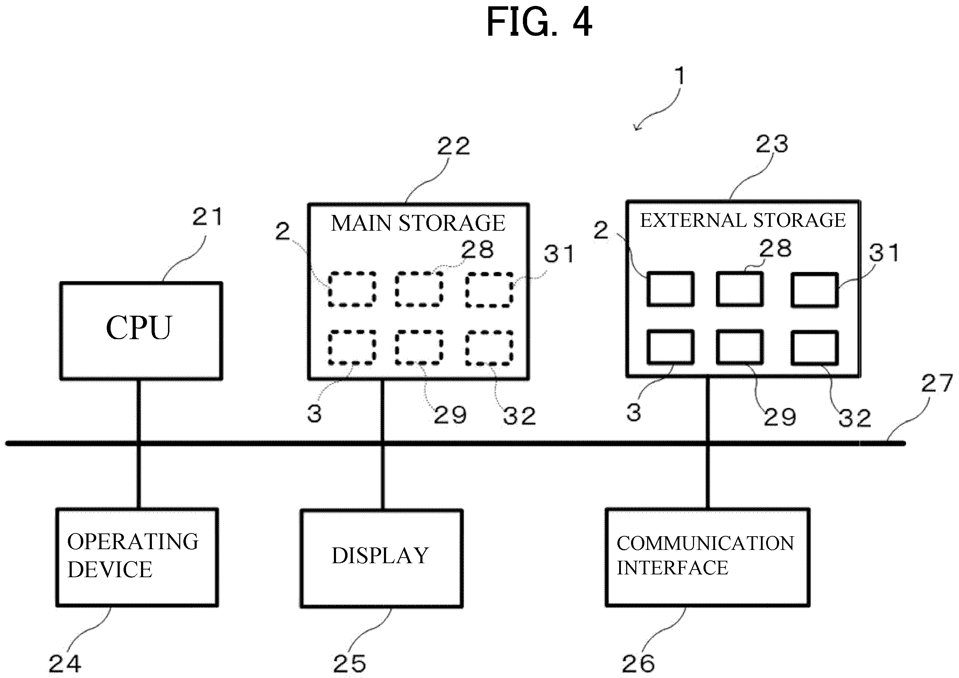

The intermolecular interaction analysis device 1 illustrated in FIG. 1 is realized, for example, by a computer having the hardware configuration illustrated in FIG. 4 executing a software program. Specifically, the intermolecular interaction analysis device 1 comprises: a central processing unit (CPU) 21 for controlling the entire device; a main storage 22 for operating as a working area for the CPU 21; an external storage 23 for storing the operation program of the CPU 21 and the like; an operating device 24; a display 25; a communication interface 26; and an internal bus 27 for connecting them.

The main storage 22 comprises a random access memory (RAM) and the like. 3D structure data 2, 3D structure data 3, an electrostatic potential evaluation program 28, an FMO calculation program 29, data indicating the charge of molecule A 31 associated with the 3D structure data 2, and data indicating the charge of molecule B 32 associated with the 3D structure data 3 are loaded from the external storage 23 into the main storage 22. The main storage 22 is also used as a working area (a temporary storage area for data) for the CPU 21.

The external storage 23 comprises a non-volatile memory such as a flash memory or a hard disk. The external storage 23 stores in advance an electrostatic potential evaluation program 28 and an FMO calculation program 29 to be executed by the CPU 21. In addition to the 3D structure data 2 and the 3D structure data 3, the external storage 23 further stores data indicating the charge of molecule A 31 and data indicating the charge of molecule B 32.

The operating device 24 comprises devices such as a keyboard and a mouse, and an interface device that connects these devices to the internal bus 27.

The display 25 comprises a cathode ray tube (CRT) and a display device such as an LCD monitor.

The communication interface 26 is an interface for transmitting and receiving data to and from external devices. The 3D structure data 2, the 3D structure data 3, the data indicating the charge of molecule A 31, the data indicating the charge of molecule B 32, and the like are transmitted and received via the communication interface 26.

Next, the intermolecular interaction analysis method realized by the operation of the intermolecular interaction analysis device according to the present embodiment 1, that is, electrostatic potential evaluation processing executed by the intermolecular interaction analysis device 1 is described.

First, the electrostatic potential evaluation program 28 and the FMO calculation program 29 are read from the external storage 23 into the main storage 22, and the CPU 21 executes the electrostatic potential evaluation program 28, thereby executing the electrostatic potential evaluation processing.

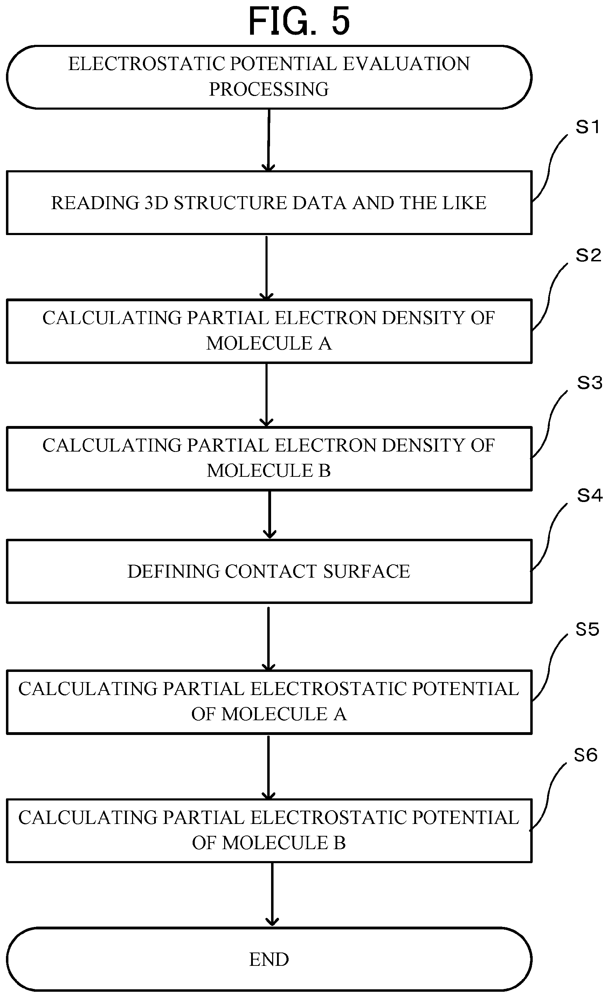

As illustrated in FIG. 5, the intermolecular interaction analysis device 1 reads the 3D structure data 2, the 3D structure data 3, the data indicating the charge of molecule A 31, and the data indicating the charge of molecule B 32 (step S1). Here, the 3D structure data 2, the 3D structure data 3, the data indicating the charge of molecule A 31, and the data indicating the charge of molecule B 32 are read from the external storage 23 into the main storage 22.

The electron density calculator 10 reads the FMO calculation program 29 from the external storage 23 into the main storage 22 and calculates the partial electron density of molecule A using the 3D structure data 2 (step S2). The data indicating the partial electron density of molecule A are stored in the main storage 22 and the external storage 23. Then, the electron density calculator 20 calculates the partial electron density of molecule B using the 3D structure data 3 (step S3). The data indicating the partial electron density of molecule B are stored in the main storage 22 and the external storage 23.

Next, the contact surface definer 30 defines the contact surface S by referring to the data indicating the partial electron density of molecule A and the data indicating the partial electron density of molecule B stored in the main storage 22 (step S4). The data indicating contact surface S are stored in the main storage 22 and the external storage 23. Then, the electrostatic potential calculator 40 refers to the FMO calculation program 29, the data indicating the contact surface S, the 3D structure data 2, and the data indicating the charge of molecule A 31 stored in the main storage 22 and calculates the partial electrostatic potential of molecule A at the contact surface S while excluding a contribution of charged amino acid residues (step S5). The data indicating the partial electrostatic potential of molecule A are stored in the main storage 22 and the external storage 23.

Furthermore, the electrostatic potential calculator 50 refers to the FMO calculation program 29, the data indicating the contact surface S, the 3D structure data 3, and the data indicating the charge of molecule B 32 stored in the main storage 22 and calculates the partial electrostatic potential of molecule B at the contact surface S while excluding a contribution of charged amino acid residues (step S6). The intermolecular interaction analysis device 1 stores the data indicating the partial electrostatic potential of molecule B in the main storage 22 and the external storage 23 and terminates the electrostatic potential evaluation processing.

In response to the input of the user's command via the operating device 24, the CPU 21 may display on the display 25 an image in which the partial electrostatic potential of molecule A and the partial electrostatic potential of molecule B are depicted on the contact surface S, or may calculate the above-described ratio $W^-$ and display it on the display 25.

The intermolecular interaction analysis device 1 according to the present embodiment calculates the partial electrostatic potential of molecule A at the contact surface S while, with the molecule A having a charge, excluding a contribution of charged amino acid residues corresponding to the charge, and calculates the partial electrostatic potential of molecule B at the contact surface S while, with the molecule B having a charge, excluding a contribution of charged amino acid residues corresponding to the charge. In this way, the effect of the total charge can be removed and the original partial electrostatic potential state at the contact surface S can be evaluated. This allows for highly accurate evaluation of intermolecular interactions such as protein-protein interactions and protein-antibody interactions.

By evaluating intermolecular interactions such as protein-protein interactions and protein-antibody interactions with high accuracy, an antibody can be designed to have a high correspondence with the electrostatic potential of a target protein, allowing efficient antibody development in a short period of time.

In addition, the contact surface definer 30 may define a surface on which the partial electron density of molecule A is equal to or greater than a reference value as the contact surface S. The surface where the partial electron density of molecule A and the partial electron density of molecule B are equal extends infinitely. Thus, it is possible to avoid defining the part where molecules A and B do not interact as the contact surface, allowing the increase in the amount of calculations to be suppressed.

Embodiment 2

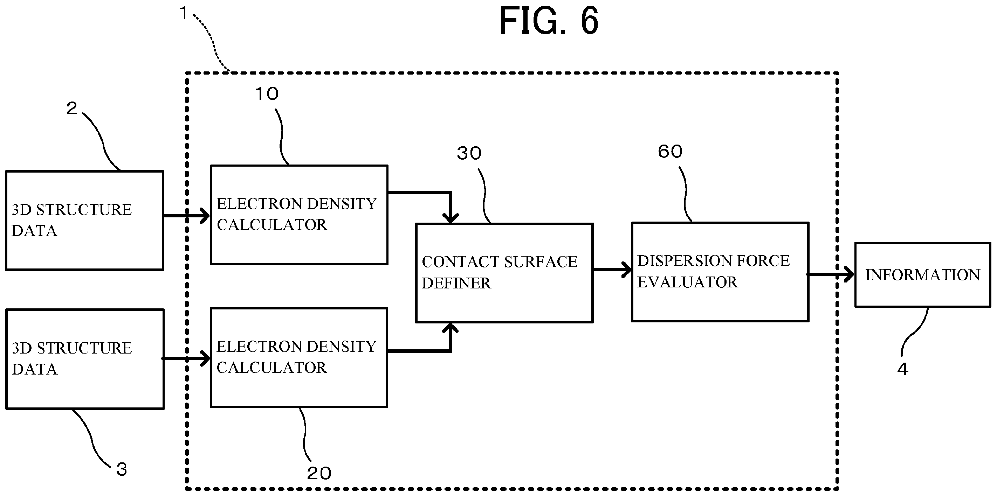

Next, Embodiment 2 of the present disclosure is described. The intermolecular interaction analysis device 5 according to the present embodiment serving as an information processor has the same hardware configuration as the intermolecular interaction analysis device 1 according to Embodiment 1 above, but has a different software configuration. The intermolecular interaction analysis device 5 comprises a dispersion force evaluator 60 in place of the electrostatic potential calculator 40 and the electrostatic potential calculator 50, as illustrated in FIG. 6.

The dispersion force evaluator 60 evaluates the dispersion force based on the partial electron density of molecule A and the partial electron density of molecule B at the contact surface S. The major component of the non-electrostatic interaction between molecules A and B is dispersion force. Since the dispersion force increases roughly in proportion to the magnitude of the induced dipole, that is, the magnitude of the overlap of the partial electron densities, the dispersion force can be evaluated by quantifying the overlap of the partial electron densities.

More specifically, the dispersion force evaluator 60 evaluates an index indicating the overlap of partial electron densities, which is the sum of the partial electron densities of molecules A and B at the contact surface S, as the dispersion force. For example, as described in Embodiment 1, in the case of calculating the electron density using 3D grid data, the dispersion force evaluator 60 calculates a value ($\rho^+$) as a sum of values x obtained by multiplying each of the partial electron density value of molecule A and the partial electron density value of molecule B in each grid lattice on the contact surface S by the infinitesimal area (the square of the length of one side of the grid) as evaluation information 4. $\rho^+$ is an index

indicating an overlap of partial electron densities.

For example, FIG. 7 illustrates $\rho^+$ at the contact surface S in the complex of PD-1 and nivolumab. The sum of $\rho^+$ at the contact surface S was 15.668. By calculating $\rho^+$, it is possible to compare $\rho^+$ between PD-1 and an antibody other than nivolumab or a modified antibody in which a specific amino acid residue of nivolumab has been substituted.

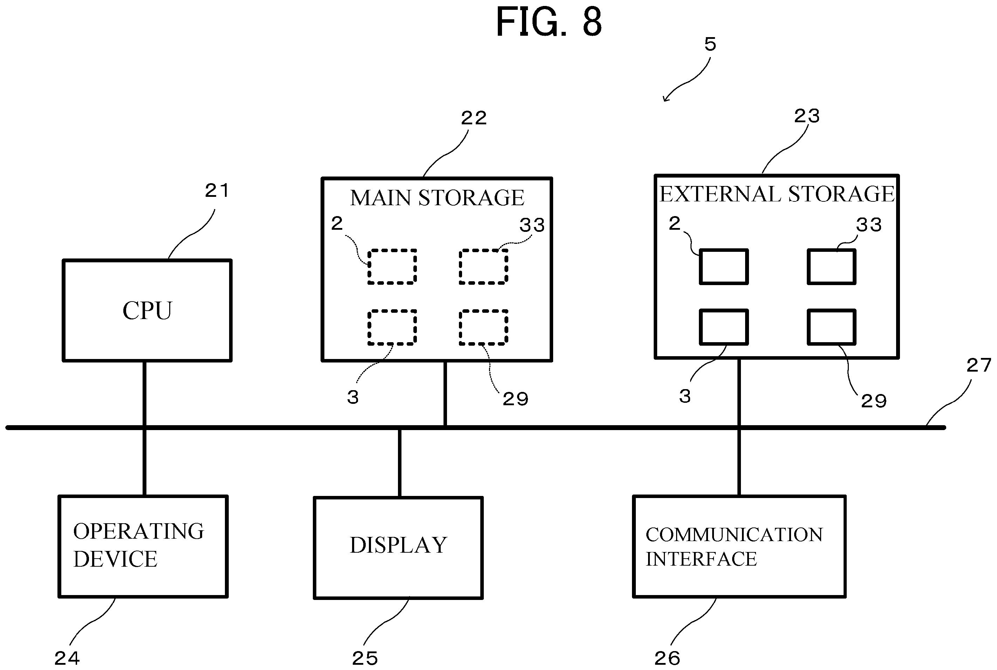

The intermolecular interaction analysis device 5 illustrated in FIG. 6 is realized, for example, by a computer having the hardware configuration illustrated in FIG. 8 executing a software program. In the main storage 22, a dispersion force evaluation program 33 is loaded from the external storage 23 in place of the electrostatic potential evaluation program 28. The external storage 23 stores in advance a dispersion force evaluation program 33 in place of the electrostatic potential evaluation program 28. The data indicating the charge of molecule A 31 and the data indicating the charge of molecule B 32 are not stored in the external storage 23.

Next, the intermolecular interaction analysis method realized by the operation of the intermolecular interaction analysis device according to the present embodiment 5, that is, dispersion force evaluation processing executed by the intermolecular interaction analysis device 5 is described.

First, the dispersion force evaluation program 33 is read from the external storage 23 into the main storage 22, and the CPU 21 executes the dispersion force evaluation program 33, thereby executing the dispersion force evaluation processing.

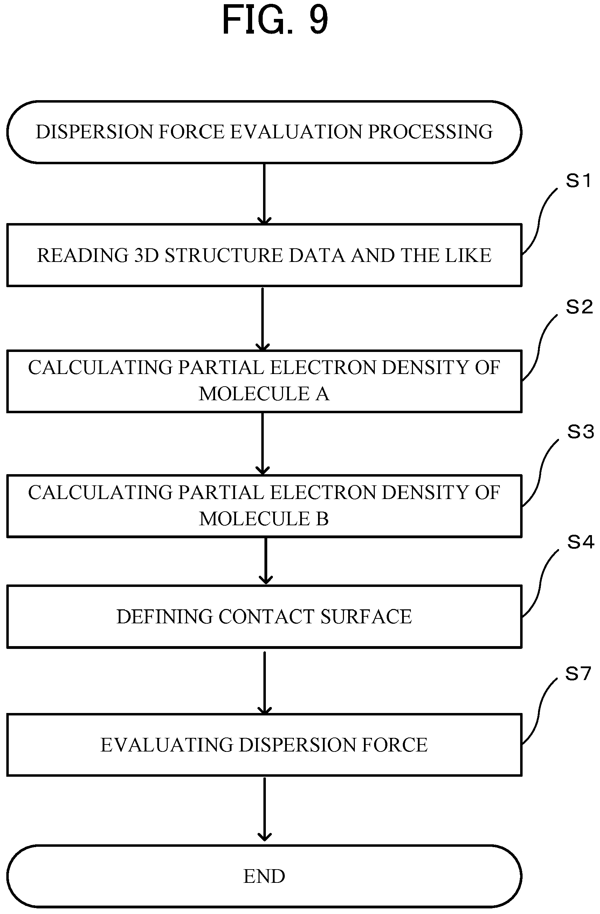

As illustrated in FIG. 9, the dispersion force evaluation processing is the same as the electrostatic potential evaluation processing in Embodiment 1 from step S1 to step S4, except that the data indicating the charge of molecule A 31 and the data indicating the charge of molecule B 32 are not read in step S1. Following step 4, the dispersion force evaluator 60 evaluates the dispersion force based on the partial electron density of molecule A and the partial electron density of molecule B at the contact surface S by referring to the data indicating contact surface S, the 3D structure data 2, the 3D structure data 3, the data indicating the partial electron density of molecule A, and the data indicating the partial electron density of molecule B stored in the main storage 22 (step S7). The intermolecular interaction analysis device 5 stores the data indicating the dispersion force in the main storage 22 and the external storage 23 and terminates the dispersion force evaluation processing.

The intermolecular interaction analysis device 5 according to the present embodiment was intended to evaluate the dispersion force based on the partial electron density of molecule A and the partial electron density of molecule B at the contact surface S. In this way, non-electrostatic interactions, which are important in PPIs, can be evaluated, and intermolecular interactions such as protein-protein interactions and protein-antibody interactions can be evaluated with high accuracy.

In response to the input of the user's command via the operating device 24, the CPU 21 may display an image of the dispersion force depicted on the contact surface S on the display 25.

The hardware configurations and software configurations of the intermolecular interaction analysis device 1 and the intermolecular interaction analysis device 5 described above are merely examples, and can be changed or modified as desired.

The core part of the processing of the intermolecular interaction analysis device 1 and the intermolecular interaction analysis device 5, which comprises the CPU 21, the main storage 22, the external storage 23, the operating device 24, the display 25, the communication interface 26, the internal bus 27, and the like, can be realized by using an ordinary computer system, without using a dedicated system. The configuration of the intermolecular interaction analysis device 1 and the intermolecular interaction analysis device 5 for executing the above-described processing may be achieved by, for example, storing a computer program of executing the above-described operations on a computer-readable recording medium (such as a flexible disk, a compact disc read-only memory (CD-ROM), or a digital versatile disk read-only memory (DVD-ROM)), distributing the recording medium, and installing the computer program on a computer. In addition, the configuration of the intermolecular interaction analysis device 1 and the intermolecular interaction analysis device 5 may be achieved by storing the computer program in a storage device of a server device on a communication network such as the Internet, and downloading the computer program by an ordinary computer system.

The intermolecular interaction analysis device 1 may further comprise a dispersion force evaluator 60. In this case, in response to the user's command, the intermolecular interaction analysis device 1 can also calculate the partial electrostatic potential of molecule A and the partial electrostatic potential of molecule B at the contact surface S or evaluate the dispersion force based on the partial electron density of molecule A and the partial electron density of molecule B at the contact surface S.

For example, in a case in which the functions of the intermolecular interaction analysis device 1 and the intermolecular interaction analysis device 5 are realized by sharing between an operating system (OS) and an application program or by cooperation between the OS and the application program, only the application program portion may be stored on a recording medium or storage device.

It is also possible for the computer program to be superimposed on a carrier wave and distributed via a communication network. For example, the computer program may be posted on a bulletin board system (BBS) on a communication network and distributed via the network. The computer program may then be started and executed under the control of the OS in the same manner as other application programs, thereby enabling the above-described processing to be performed.

According to Embodiments 1 and 2 above, the binding affinity (binding strength) of PPI can be evaluated by quantifying electrostatic complementarity. The way the intricately distributed positive and negative charges fit together like a puzzle indicates a highly specific interaction, making it possible to evaluate the specificity of PPI, which was difficult using previous computational techniques. Examples of the applied use of the present technique include analyzing the contribution of each amino acid in the variable region to affinity and specificity for a target protein as well as designing an antibody or the like such that it corresponds closely to the electrostatic potential of a target protein with the aim of improving affinity and specificity, improving antibody stability, and the like.

The foregoing describes some example embodiments for explanatory purposes. Although the foregoing discussion has presented specific embodiments, persons skilled in the art will recognize that changes may be made in form and detail without departing from the broader spirit and scope of the invention. Accordingly, the specification and drawings are to be regarded in an illustrative rather than a restrictive sense. This detailed description, therefore, is not to be taken in a limiting sense, and the scope of the invention is defined only by the included claims, along with the full range of equivalents to which such claims are entitled.

This application claims the benefit of Japanese Patent Application No. 2022-139395, filed on September 1, 2022, the entire disclosure of which is incorporated by reference herein.

Industrial Applicability

The present disclosure is useful for analyzing protein-protein or protein-antibody interactions.

Reference Signs List

| | |
|---|---|
| 1, 5 | Intermolecular interaction analysis device |
| 2, 3 | 3D structure data |
| 4 | Evaluation information |
| 10, 20 | Electron density calculator |
| 30 | Contact surface definer |
| 40, 50 | Electrostatic potential calculator |
| 60 | Dispersion force evaluator |
| 21 | CPU |
| 22 | Main storage |
| 23 | External storage |
| 24 | Operating device |
| 25 | Display |
| 26 | Communication interface |
| 27 | Internal bus |
| 28 | Electrostatic potential evaluation program |
| 29 | FMO calculation program |
| 31 | Data showing the charge of molecule A |
| 32 | Data showing the charge of molecule B |
| 33 | Dispersion force evaluation program |

**Claims**

**1.** An intermolecular interaction analysis device comprising:

a first electron density calculator that calculates a partial electron density of a first molecule containing a plurality of amino acid residues linked via peptide bonds regarding 3D structure data of the first molecule by a fragment molecular orbital method;
a second electron density calculator that calculates a partial electron density of a second molecule containing a plurality of amino acid residues linked via peptide bonds, the second molecule being arranged at a position interacting with the first molecule relative to a position of the first molecule, regarding 3D structure data of the second molecule by a fragment molecular orbital method;
a contact surface definer that defines a surface where the partial electron density of the first molecule and the partial electron density of the second molecule are equal as a contact surface;

a first electrostatic potential calculator that calculates a partial electrostatic potential of the first molecule at the contact surface by a fragment molecular orbital method while, with the first molecule having a charge, excluding a contribution of charged amino acid residues corresponding to the charge of the first molecule; and

a second electrostatic potential calculator that calculates a partial electrostatic potential of the second molecule at the contact surface by a fragment molecular orbital method while, with the second molecule having a charge, excluding a contribution of charged amino acid residues corresponding to the charge of the second molecule.

2. The intermolecular interaction analysis device according to claim 1, wherein the contact surface definer defines a surface where the partial electron density of the first molecule is equal to or greater than a reference value as the contact surface.

3. The intermolecular interaction analysis device according to claim 1 or 2, wherein the first electrostatic potential calculator,

with the first molecule having a positive charge of +n, selects n residues among arginine residues and lysine residues contained in the first molecule as the charged amino acid residues in descending order of distance from the contact surface, and calculates a partial electrostatic potential of the first molecule as a whole while excluding a partial electrostatic potential of the selected charged amino acid residues, or

with the first molecule having a negative charge of -n, selects n residues among aspartic acid and glutamic acid residues contained in the first molecule as the charged amino acid residues in descending order of distance from the contact surface, and calculates a partial electrostatic potential of the first molecule as a whole while excluding a partial electrostatic potential of the selected charged amino acid residues, and

wherein the second electrostatic potential calculator,

with the second molecule having a positive charge of +n, selects n residues among arginine residues and lysine residues contained in the second molecule as the charged amino acid residues in descending order of distance from the contact surface, and calculates a partial electrostatic potential of the second molecule as a whole while excluding a partial electrostatic potential of the selected charged amino acid residues, or

with the second molecule having a negative charge of -n, selects n residues among aspartic acid and glutamic acid residues contained in the second molecule as the charged amino acid residues in descending order of distance from the contact surface, and calculates a partial electrostatic potential of the second molecule as a whole while excluding a partial electrostatic potential of the selected charged amino acid residues.

4. An intermolecular interaction analysis device comprising:

a first electron density calculator that calculates a partial electron density of a first molecule containing a plurality of amino acid residues linked via peptide bonds regarding 3D structure data of the first molecule by a fragment molecular orbital method;

a second electron density calculator that calculates a partial electron density of a second molecule containing a plurality of amino acid residues linked via peptide bonds, the second molecule being arranged at a position interacting with the first molecule relative to a position of the first molecule, regarding 3D structure data of the second molecule by a fragment molecular orbital method;

a contact surface definer that defines a surface where the partial electron density of the first molecule and the partial electron density of the second molecule are equal as a contact surface; and

a dispersion force evaluator that evaluates dispersion force based on a partial electron density of the first molecule and a partial electron density of the second molecule at the contact surface.

5. The intermolecular interaction analysis device according to claim 4, wherein the contact surface definer defines a surface where the partial electron density of the first molecule is equal to or greater than a reference value as the contact surface.

6. The intermolecular interaction analysis device according to claim 4 or 5, wherein the dispersion force evaluator evaluates an index indicating an overlap of partial electron densities calculated from a sum of the partial electron density of the first molecule and the partial electron density of the second molecule at the contact surface as the dispersion force.

7. An intermolecular interaction analysis device comprising:

a first electron density calculator that calculates a partial electron density of a first molecule containing a plurality of amino acid residues linked via peptide bonds regarding 3D structure data of the first molecule by a fragment molecular orbital method;

a second electron density calculator that calculates a partial electron density of a second molecule containing a plurality of amino acid residues linked via peptide bonds, the second molecule being arranged at a position interacting with the first molecule relative to a position of the first molecule, regarding 3D structure data of the second molecule by a fragment molecular orbital method;

a contact surface definer that defines a surface where the partial electron density of the first molecule and the partial electron density of the second molecule are equal as a contact surface;

a first electrostatic potential calculator that calculates a partial electrostatic potential of the first molecule at the contact surface by a fragment molecular orbital method while, with the first molecule having a charge, excluding a contribution of charged amino acid residues corresponding to the charge of the first molecule;

a second electrostatic potential calculator that calculates a partial electrostatic potential of the second molecule at the contact surface by a fragment molecular orbital method while, with the second molecule having a charge, excluding a contribution of charged amino acid residues corresponding to the charge of the second molecule; and

a dispersion force evaluator that evaluates dispersion force based on a partial electron density of the first molecule and a partial electron density of the second molecule at the contact surface.

8.  An intermolecular interaction analysis method comprising:

calculating a partial electron density of a first molecule containing a plurality of amino acid residues linked via peptide bonds regarding 3D structure data of the first molecule by a fragment molecular orbital method;

calculating a partial electron density of a second molecule containing a plurality of amino acid residues linked via peptide bonds, the second molecule being arranged at a position interacting with the first molecule relative to a position of the first molecule, regarding 3D structure data of the second molecule by a fragment molecular orbital method;

defining a surface where the partial electron density of the first molecule and the partial electron density of the second molecule are equal as a contact surface;

calculating a partial electrostatic potential of the first molecule at the contact surface by a fragment molecular orbital method while, with the first molecule having a charge, excluding a contribution of charged amino acid residues corresponding to the charge of the first molecule; and

calculating a partial electrostatic potential of the second molecule at the contact surface by a fragment molecular orbital method while, with the second molecule having a charge, excluding a contribution of charged amino acid residues corresponding to the charge of the second molecule.

9.  An intermolecular interaction analysis method comprising:

calculating a partial electron density of a first molecule containing a plurality of amino acid residues linked via peptide bonds regarding 3D structure data of the first molecule by a fragment molecular orbital method;

calculating a partial electron density of a second molecule containing a plurality of amino acid residues linked via peptide bonds, the second molecule being arranged at a position interacting with the first molecule relative to a position of the first molecule, regarding 3D structure data of the second molecule by a fragment molecular orbital method;

defining a surface where the partial electron density of the first molecule and the partial electron density of the second molecule are equal as a contact surface; and

evaluating dispersion force based on a partial electron density of the first molecule and a partial electron density of the second molecule at the contact surface.

10. An intermolecular interaction analysis method comprising:

calculating a partial electron density of a first molecule containing a plurality of amino acid residues linked via peptide bonds regarding 3D structure data of the first molecule by a fragment molecular orbital method;

calculating a partial electron density of a second molecule containing a plurality of amino acid residues linked via peptide bonds, the second molecule being arranged at a position interacting with the first molecule relative to a position of the first molecule, regarding 3D structure data of the second molecule by a fragment molecular orbital method;

defining a surface where the partial electron density of the first molecule and the partial electron density of the second molecule are equal as a contact surface;

calculating a partial electrostatic potential of the first molecule at the contact surface by a fragment molecular orbital method while, with the first molecule having a charge, excluding a contribution of charged amino acid residues corresponding to the charge of the first molecule;

calculating a partial electrostatic potential of the second molecule at the contact surface by a fragment molecular orbital method while, with the second molecule having a charge, excluding a contribution of charged amino acid residues corresponding to the charge of the second molecule; and

evaluating dispersion force based on a partial electron density of the first molecule and a partial electron density of the second molecule at the contact surface.

11. A program of allowing a computer to function as:

a first electron density calculator that calculates a partial electron density of a first molecule containing a plurality of amino acid residues linked via peptide bonds regarding 3D structure data of the first molecule by a fragment molecular orbital method;

a second electron density calculator that calculates a partial electron density of a second molecule containing a plurality of amino acid residues linked via peptide bonds, the second molecule being arranged at a position interacting with the first molecule relative to a position of the first molecule, regarding 3D structure data of the second molecule by a fragment molecular orbital method;

a contact surface definer that defines a surface where the partial electron density of the first molecule and the partial electron density of the second molecule are equal as a contact surface;

a first electrostatic potential calculator that calculates a partial electrostatic potential of the first molecule at the contact surface by a fragment molecular orbital method while, with the first molecule having a charge, excluding a contribution of charged amino acid residues corresponding to the charge of the first molecule; and

a second electrostatic potential calculator that calculates a partial electrostatic potential of the second molecule at the contact surface by a fragment molecular orbital method while, with the second molecule having a charge, excluding a contribution of charged amino acid residues corresponding to the charge of the second molecule.

12. A program of allowing a computer to function as:

a first electron density calculator that calculates a partial electron density of a first molecule containing a plurality of amino acid residues linked via peptide bonds regarding 3D structure data of the first molecule by a fragment molecular orbital method;

a second electron density calculator that calculates a partial electron density of a second molecule containing a plurality of amino acid residues linked via peptide bonds, the second molecule being arranged at a position interacting with the first molecule relative to a position of the first molecule, regarding 3D structure data of the second molecule by a fragment molecular orbital method;

a contact surface definer that defines a surface where the partial electron density of the first molecule and the partial electron density of the second molecule are equal as a contact surface; and

a dispersion force evaluator that evaluates dispersion force based on a partial electron density of the first molecule and a partial electron density of the second molecule at the contact surface.

13. A program of allowing a computer to function as:

a first electron density calculator that calculates a partial electron density of a first molecule containing a plurality of amino acid residues linked via peptide bonds regarding 3D structure data of the first molecule by a fragment molecular orbital method;

a second electron density calculator that calculates a partial electron density of a second molecule containing a plurality of amino acid residues linked via peptide bonds, the second molecule being arranged at a position interacting with the first molecule relative to a position of the first molecule, regarding 3D structure data of the second molecule by a fragment molecular orbital method;

a contact surface definer that defines a surface where the partial electron density of the first molecule and the partial electron density of the second molecule are equal as a contact surface; and

a first electrostatic potential calculator that calculates a partial electrostatic potential of the first molecule at the contact surface by a fragment molecular orbital method while, with the first molecule having a charge, excluding a contribution of charged amino acid residues corresponding to the charge of the first molecule;

a second electrostatic potential calculator that calculates a partial electrostatic potential of the second molecule at the contact surface by a fragment molecular orbital method while, with the second molecule having a charge, excluding a contribution of charged amino acid residues corresponding to the charge of the second molecule; and

a dispersion force evaluator that evaluates dispersion force based on a partial electron density of the first molecule and a partial electron density of the second molecule at the contact surface.

14. An intermolecular interaction analysis device comprising:

a memory that stores a program; and
a central processing unit (CPU) that reads and executes the program from the memory,
wherein the CPU
calculates a partial electron density of a first molecule containing a plurality of amino acid residues linked via peptide bonds regarding 3D structure data of the first molecule by a fragment molecular orbital method,
calculates a partial electron density of a second molecule containing a plurality of amino acid residues linked via peptide bonds, the second molecule being arranged at a position interacting with the first molecule relative to a position of the first molecule, regarding 3D structure data of the second molecule by a fragment molecular orbital method,
defines a surface where the partial electron density of the first molecule and the partial electron density of the second molecule are equal as a contact surface,
calculates a partial electrostatic potential of the first molecule at the contact surface by a fragment molecular orbital method while, with the first molecule having a charge, excluding a contribution of charged amino acid residues corresponding to the charge of the first molecule, and
calculates a partial electrostatic potential of the second molecule at the contact surface by a fragment molecular orbital method while, with the second molecule having a charge, excluding a contribution of charged amino acid residues corresponding to the charge of the second molecule.

15. An intermolecular interaction analysis device comprising:

a memory that stores a program; and
a central processing unit (CPU) that reads and executes the program from the memory,
wherein the CPU
calculates a partial electron density of a first molecule containing a plurality of amino acid residues linked via peptide bonds regarding 3D structure data of the first molecule by a fragment molecular orbital method,
calculates a partial electron density of a second molecule containing a plurality of amino acid residues linked via peptide bonds, the second molecule being arranged at a position interacting with the first molecule relative to a position of the first molecule, regarding 3D structure data of the second molecule by a fragment molecular orbital method,
defines a surface where the partial electron density of the first molecule and the partial electron density of the second molecule are equal as a contact surface, and
evaluates dispersion force based on a partial electron density of the first molecule and a partial electron density of the second molecule at the contact surface.

16. An intermolecular interaction analysis device comprising:

a memory that stores a program; and
a central processing unit (CPU) that reads and executes the program from the memory,
wherein the CPU
calculates a partial electron density of a first molecule containing a plurality of amino acid residues linked via peptide bonds regarding 3D structure data of the first molecule by a fragment molecular orbital method,
calculates a partial electron density of a second molecule containing a plurality of amino acid residues linked via peptide bonds, the second molecule being arranged at a position interacting with the first molecule relative to a position of the first molecule, regarding 3D structure data of the second molecule by a fragment molecular orbital method,
defines a surface where the partial electron density of the first molecule and the partial electron density of the second molecule are equal as a contact surface,
calculates a partial electrostatic potential of the first molecule at the contact surface by a fragment molecular orbital method while, with the first molecule having a charge, excluding a contribution of charged amino acid residues corresponding to the charge of the first molecule,
calculates a partial electrostatic potential of the second molecule at the contact surface by a fragment molecular orbital method while, with the second molecule having a charge, excluding a contribution of charged amino acid residues corresponding to the charge of the second molecule, and

evaluates dispersion force based on a partial electron density of the first molecule and a partial electron density of the second molecule at the contact surface.

# FIG. 1

Diagram showing: 3D STRUCTUR DATA (2) → ELECTRON DENSITY CALCULATOR (10); 3D STRUCTUR DATA (3) → ELECTRON DENSITY CALCULATOR (20). Both electron density calculators feed into CONTACT SURFACE DEFINER (30), which outputs to ELECTROSTATIC POTENTIAL CALCULATOR (40) and ELECTROSTATIC POTENTIAL CALCULATOR (50), both feeding into EVALUATION INFORMATION (4). The dashed box encloses system (1).

# FIG. 2

# FIG. 3A

-0.1  -0.05  0.0  +0.05  +0.1

# FIG. 3B

-0.1  -0.05  0.0  +0.05  +0.1

# FIG. 4

EP 4 567 816 A1

# FIG. 5

```
┌─────────────────────────────────────────────────┐
│   ELECTROSTATIC POTENTIAL EVALUATION             │
│            PROCESSING                            │
└─────────────────────────────────────────────────┘
                      │
                      ▼                              S1
┌─────────────────────────────────────────────────┐
│      READING 3D STRUCTURE DATA AND THE LIKE      │
└─────────────────────────────────────────────────┘
                      │
                      ▼                              S2
┌─────────────────────────────────────────────────┐
│    CALCULATING PARTIAL ELECTRON DENSITY OF       │
│               MOLECULE A                         │
└─────────────────────────────────────────────────┘
                      │
                      ▼                              S3
┌─────────────────────────────────────────────────┐
│    CALCULATING PARTIAL ELECTRON DENSITY OF       │
│               MOLECULE B                         │
└─────────────────────────────────────────────────┘
                      │
                      ▼                              S4
┌─────────────────────────────────────────────────┐
│           DEFINING CONTACT SURFACE               │
└─────────────────────────────────────────────────┘
                      │
                      ▼                              S5
┌─────────────────────────────────────────────────┐
│  CALCULATING PARTIAL ELECTROSTATIC POTENTIAL     │
│             OF MOLECULE A                         │
└─────────────────────────────────────────────────┘
                      │
                      ▼                              S6
┌─────────────────────────────────────────────────┐
│  CALCULATING PARTIAL ELECTROSTATIC POTENTIAL     │
│             OF MOLECULE B                         │
└─────────────────────────────────────────────────┘
                      │
                      ▼
┌─────────────────────────────────────────────────┐
│                    END                           │
└─────────────────────────────────────────────────┘
```

# FIG. 6

# FIG. 7

-0.005          0.0          +0.005

# FIG. 8

EP 4 567 816 A1

# FIG. 9

```
┌─────────────────────────────────────────────────┐
│   DISPERSION FORCE EVALUATION PROCESSING          │
└─────────────────────────────────────────────────┘
                        │
                        ▼
┌─────────────────────────────────────────────────┐  S1
│      READING 3D STRUCTURE DATA AND THE LIKE       │
└─────────────────────────────────────────────────┘
                        │
                        ▼
┌─────────────────────────────────────────────────┐  S2
│      CALCULATING PARTIAL ELECTRON DENSITY OF      │
│                   MOLECULE A                       │
└─────────────────────────────────────────────────┘
                        │
                        ▼
┌─────────────────────────────────────────────────┐  S3
│      CALCULATING PARTIAL ELECTRON DENSITY OF      │
│                   MOLECULE B                       │
└─────────────────────────────────────────────────┘
                        │
                        ▼
┌─────────────────────────────────────────────────┐  S4
│             DEFINING CONTACT SURFACE              │
└─────────────────────────────────────────────────┘
                        │
                        ▼
┌─────────────────────────────────────────────────┐  S7
│           EVALUATING DISPERSION FORCE             │
└─────────────────────────────────────────────────┘
                        │
                        ▼
┌─────────────────────────────────────────────────┐
│                     END                            │
└─────────────────────────────────────────────────┘
```

**EP 4 567 816 A1**

<div align="center">

**INTERNATIONAL SEARCH REPORT**

</div>

| | International application No. |
|---|---|
| | **PCT/JP2023/030775** |

**A.    CLASSIFICATION OF SUBJECT MATTER**

*G16B 15/30*(2019.01)i
FI:    G16B15/30

According to International Patent Classification (IPC) or to both national classification and IPC

**B.    FIELDS SEARCHED**

Minimum documentation searched (classification system followed by classification symbols)

G16B15/30

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

**C.    DOCUMENTS CONSIDERED TO BE RELEVANT**

| Category* | Citation of document, with indication, where appropriate, of the relevant passages | Relevant to claim No. |
|---|---|---|
| A | WO 2013/069348 A1 (JAPAN AS REPRESENTED BY DIRECTOR GENERAL OF NATIONAL INSTITUTE OF HEALTH SCIENCES) 16 May 2013 (2013-05-16)<br>entire text, all drawings | 1-16 |
| A | MONTELEONE, Stefania et al. Hotspot Identification and Drug Design of Protein-Protein Interaction Modulators Using the Fragment Molecular Orbital Method. Journal of Chemical Information and Modeling, vol. 62, Issue 16, [online], 22 August 2022, pp. 3784-3799, Internet:<URL:https://pubs.acs.org/doi/full/10.1021/acs.jcim.2c00457>[retrieved on 30 October 2023]<br>entire text, all drawings | 1-16 |

☐ Further documents are listed in the continuation of Box C.　　　☑ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **30 October 2023** | **07 November 2023** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (January 2015)

## INTERNATIONAL SEARCH REPORT
### Information on patent family members

International application No.

**PCT/JP2023/030775**

| Patent document cited in search report | Publication date (day/month/year) | Patent family member(s) | Publication date (day/month/year) |
|---|---|---|---|
| WO 2013/069348 A1 | 16 May 2013 | US 2014/0372047 A1 entire text, all drawings | |

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2019235567 A

- JP 2022139395 A

**Non-patent literature cited in the description**

- **H. OZONO** ; **T. ISHIKAWA**. Visualization of the Interfacial Electrostatic Complementarity: A Method for Analysis of Protein-Protein Interaction Based on Ab Initio Quantum Chemical Calculations. *J Chem. Theory Comput.*, 2021, vol. 17, 5600-5610